# EUROPEAN PATENT SPECIFICATION

(11) **EP 3 171 845 B1**
(45) Date of publication and mention of the grant of the patent: **03.10.2018**
(21) Application number: 15879279.6
(22) Date of filing: 11.11.2015
(51) Int. Cl.: A61H 31/02, A61M 16/00, A61M 16/06, A61M 16/08

(54) **VENTILATION MASK**
BEATMUNGSMASKE
MASQUE DE VENTILATION

(30) Priority: 27.01.2015 AU 2015900220
(43) Date of publication of application: 31.05.2017
(73) Proprietor: International Health Group Pty Ltd., Sydney, New South Wales 2072 (AU)
(72) Inventor: RITZ, Gavin, Sydney, New South Wales 2072 (AU); RITZ, Deborah, Sydney, New South Wales 2072 (AU)
(74) Representative: Westphal, Mussgnug & Partner Patentanwälte mbB
(86) International application number: PCT/AU2015/050702
(87) International publication number: WO 2016/119006

(56) References cited:
- WO-A1-2013/171624
- DE-A1- 3 331 374
- GB-A- 2 336 547
- GB-A- 2 336 547
- US-A- 4 022 200
- US-A- 4 770 169
- US-A- 4 770 169

## Description

### Technical Field

The present invention relates to masks for delivering air or other gases to patients, particularly but not exclusively neonates.

### Background of the Invention

Masks are used in various clinical settings, such as hospitals, to deliver air, oxygen or other medical gases to patients. In some instances, they are a relatively loose fit and are not required to maintain a seal against the face.

However, in many instances it is desirable that a relatively tight fit is maintained. This is particularly the case where a gas is supplied under pressure to the patients, for example in a resuscitation situation.

For example, neonatal resuscitation typically takes place using a relatively sophisticated, controlled source of pressurised air and/or oxygen. This may include positive end expiratory pressure and Peak Inspiratory Pressure (PEEP and PIP) to assist in correct lung inflation and improve blood oxygenation. It is understood in the art that it is critical to deliver air at the correct pressure, volume, gas mix and breathing rate, so that the neonate's lungs are not damaged, whilst an effective air flow to assist resuscitation is provided.

However, in order to achieve the desired outcome for the neonate, it is necessary that this air is effectively delivered through the mask in the desired way. If the mask is not relatively well sealed to the infant's mouth and nose, much of the air will leak out, and the desired delivery of air will not occur. This is a common situation in conventional practice.

The best practice for infant resuscitation is to hold the mask in the correct position on the face, whilst also supporting the neonate's head, and placing the neonate in the correct posture. This is a rather difficult task in practice, even with two practitioners assisting. It will be appreciated that the special needs and delicacy of a neonate necessitate the utmost care be taken in this procedure.

In other contexts, for example delivery of medical gases to adults, it is usual to provide straps or similar arrangements to hold the mask in place. In some cases the mask may be over the mouth alone, or both the mouth and nose. However, these provide only a general retention force, and so do not assist the seal directly. Also, some users find the straps restrictive and even claustrophobic.

GB patent application No 2336547 by Gee discloses a mask with a double walled structure, in which the space between the walls surrounding the central chamber is evacuated to provide a suction seal around the periphery of the mask. It is an object of the present invention to an improved mask for delivering medical gases to patients.

### Summary of the Invention

The invention is defined by the appended claims.

In a first broad form, the present invention provides a mask which is adapted to be connected to a source of suction, so as to provide a lowered pressure area to retain the mask on the face of the patient. In this way, the mask can be held in place while a medical gas is delivered through the mask.

According to one aspect, the present invention provides a mask for the delivery of a medical gas under positive pressure, the mask including a single shell forming a chamber having an outlet, a first passage connecting the chamber to the exterior of the mask, a sealing recess isolated from the chamber and open to allow engagement with the face of a user, and extending around the periphery of the outlet, and a second passage connecting the sealing recess to the exterior of the mask, the arrangement being such that operatively the mask may be placed on a user so as to cover the airway, suction may be connected to the second passage so that the sealing recess seals the mask against the face of the user, and a medical gas may be supplied to the first passage for delivery through the outlet to the airway of the user.

According to another aspect, the present invention provides a mask for the delivery of a medical gas including a single shell forming a chamber having an outlet, a first passage connecting the chamber to the exterior of the mask, a sealing recess isolated from the chamber and open to allow engagement with the face of a user, and a second passage connecting the sealing recess to the exterior of the mask, the arrangement being such that operatively the mask may be placed on the user so as to cover the airway, suction may be connected to the second passage so that the sealing recess seals the mask against the face of the user, and a medical gas may be supplied to the first passage for delivery through the outlet to the airway of user.

According to another aspect, the present disclosure provides method for retaining a mask against n the face of a user, so that a medical gas may be delivered under positive pressure, including:
(a) providing a mask including a chamber with an outlet, a sealing recess isolated from the chamber, open to the interior of the mask, and extending around the periphery of the outlet;
(b) connecting the chamber to a source of medical gas and the sealing recess to a suction system,
(c) placing the mask over an airway of a user;
(d) operating the suction system so that the mask is retained against the face, so that the medical gas may be delivered.

Thus, implementations of the present invention allow for a mask to be retained on the face of a user, without requiring any additional devices to be attached around the head of the user. In many applications, it able to provide a simpler and effective attachment to the user. Moreover, as in many medical settings a suction facility is provided, there is no additional cost or inconvenience to the practitioner or hospital to utilise a device according to an implementation of the present invention.

### Brief Description of the Drawings

An illustrative implementation of the present invention will now be described with reference to the accompanying figures in which:
Figure 1 shows an isometric view of one implementation of the present invention;
Figure 2 shows a bottom view of the implementation of figure 1;

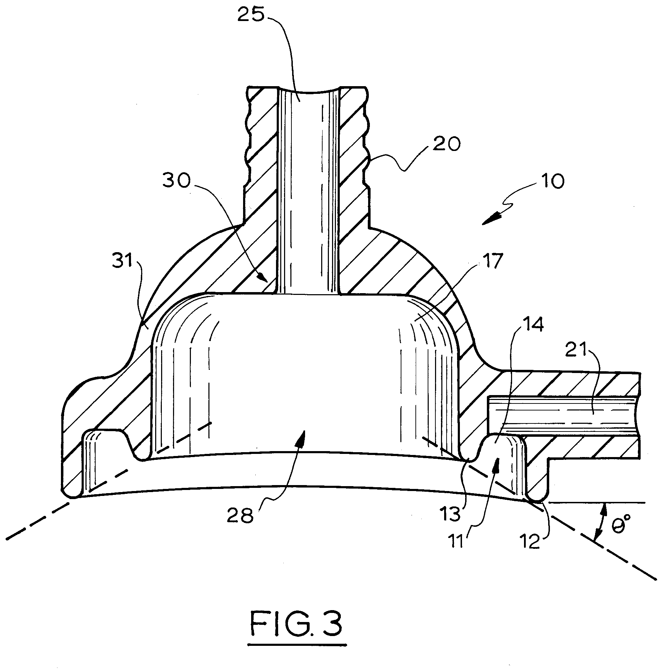

Figure 3 is a cross sectional view of the implementation of figure 1; and
Figure 4 is an illustration of a notional operational device on an infant.

### Detailed Description of the invention

The present invention will be described with reference to specific examples, which are to be understood as illustrative of the scope of the present invention and not limitative of the scope thereof. It will be appreciated that there are many different alternative implementations possible using the underlying concepts of the present invention.

For the purpose of the specification and claims, the term medical gas is intended to be interpreted broadly. It encompasses air, oxygen, or a mixture thereof, either for resuscitation, CPAP, PEEP, PIP or any other purpose. It also encompasses other gases, for example for the delivery of different gas mixtures, therapeutic agents, anaesthetics, pharmaceuticals, or other agents, either alone or mixed with air. The gases may be either at ambient or another temperature. The gases may be delivered at a relatively low pressure, for example as supplementary oxygen, or at a higher pressure, for example for resuscitation. The gas may have particles or droplet entrained or mixed therein. The present invention is not limited in scope to any particular gas or treatment.

The main implementation to be described relates to a neonatal resuscitation system. However, the present invention may be applied to any situation where a medical gas is required to be delivered, and the mask is required to be correctly placed on the airways of a user. This includes, for example, resuscitation and ventilation systems, delivery of anaesthetics, delivery of gas carried treatments (for example for respiratory conditions), treatments for sleep disorders such as CPAP (continuous positive airway pressure) devices, and any other application where correct attachment of the mask around the airways may be useful.

The transition from foetal to extrauterine life is characterised by a series of unique physiological events. Among these, the lungs change from fluid-filled to air-filled, pulmonary blood flow increases dramatically, and intracardiac and extracardiac shunts initially reverse direction and subsequently close. Normal term newborns exert negative pressures as high as about -8.0 kPa when starting to expand their lungs.

For the first few breaths, these pressures are greater than those needed for subsequent breaths. Likewise, in those newborns that need assistance to initiate lung expansion, the fluid-filled alveoli may require higher peak inspiratory and end expiratory pressures than those commonly used in subsequent ventilation, or in resuscitation later in infancy.

There are significant differences in the respiratory systems of neonates, children and adults. Neonates are not small children and children are not small adults. The anatomy of an infant, head large, neck short, tongue large, narrow nasal passages which obstruct easily, larynx more cephalad (C4) and anterior, cricoid cartilage narrowest part of airway, epiglottis long and stiff, trachea short (∼5cm in newborns). The small radius of the trachea causes an increase in resistance to flow in the trachea; further, inflammation or secretions in the airway cause an exaggerated degree of obstruction in infants, and this is commonly found with neonates requiring resuscitation.

At birth the alveoli are thick walled and only number 10% of the adult total. Lung growth occurs by alveolar multiplication until 6 - 8 years. The airways remain relatively narrow until then. Ventilation is almost entirely diaphragmatic. Infant lungs have poor elastic properties. It takes some 2 years of life until the geometry of the rib cage changes, with the gradual development of the "bucket handle" configuration seen in the adult. Ribs tend to be more horizontal in infants and this limits the potential for thoracic expansion. (http://www.aic.cuhk.edu.hk/web8/Paediatric%20anatomy%20&%20physiology.htm)

Further, in terms of the biomechanics of ventilation, the chest wall compliance of infant is very high, and drops as they grow. In contrast, lung compliance is very low at birth and steadily increases through puberty. FRC of newborns are maintained through high respiratory rate, controlled expiration (laryngeal braking), and the tonic activity of ventilatory muscles.

The physiological dead space is approx. 30% of the tidal volume, as in adults, but the absolute volume is small, so that any increase caused by apparatus (mask or equipment) deadspace has a proportionally greater effect on infants. The problem is further exacerbated with premature neonates with their lungs (with significantly more problems) and body size being even smaller than full term newborns.

The newborn requiring resuscitation is very dependent on accurate delivery of air pressure and flow with almost no tolerance for variability of these factors, (adult tolerances are significantly higher and they can survive with much higher variability). In other words the requirements for accuracy of ventilation are significantly more demanding for neonates than adults.

There have been very large increases in efficiency and effectiveness in neonatal resuscitation in recent years, with sophisticated resuscitation equipment producing very accurate delivery flows and pressures to the neonate. Resuscitation machines are used in labour and delivery wards, and neonatal Intensive care units (NICU) worldwide. Current neonatal resuscitation guidelines recommend the use of a T-piece device (part of the equipment delivery systems) with the potential of achieving controlled targeted Peak Inspiratory pressures (PIP) and delivering consistent Positive End Expiratory Pressure (PEEP) to help Functional Residual Capacity (FRC) and improve lung volume in the neonate. Target PEEP pressure are generally 0.5 kPa and PIP 2.5 kPa.

The T-piece connector in these systems connects onto special sized neonatal resuscitation masks. Air flows can range between 5 l/min and 15 l/min at specified pressures. T-piece circuits also typically include valves than can control the timing and flows of PEEP and PIP pressures.

Special sized and shaped masks have been designed and made to accompany neonatal resuscitation equipment. These masks are specifically designed to conform comfortably to an infant's face, facilitating an anatomical seal for the purposes of resuscitation and to meet all the other critical respiratory requirements mentioned above.

However these masks are required to fit the neonates face and make (ideally) 100% seals on the neonate's skin for the accurate delivery of PIP and PEEP pressures, no dead space and accurate flow rates. Due to the accurate pressures required mask leakage is the single biggest problem in neonatal resuscitation. In some studies, 14% leakage is found with experienced operators.

Prior art neonatal resuscitation masks require a hand technique to seal, it's very difficult to maintain the seal and keep the baby's head in the correct position (called the sniffing position) to facilitate open airways for lung inflation. The technique requires both pushing and pulling which is very trying under life and death situations.

The implementation of the present invention to be discussed below includes a mask structure (shape, material, flexibility, hardness) and mask function (gas flow, gas pressure, face adherence, suction pressure and flow) intended to conform to newborn structure (anatomy of head, respiratory system) and newborn function (physiology of respiratory system). Due to the design of the mask the required head positioning (sniffing position) is now not required as the mask design has facilitated such accurate delivery of pressures and flows that even slightly restricted airways are not problematic.

It will be understood that the requirements for adult resuscitation and other uses are significantly different from the neonatal application discussed in detail. However, the general principle may be applied to other applications, for example in adults, with suitable modifications to structures, materials, dimensions and other characteristics.

The present invention has particular advantages where a positive pressure is required to be delivered, as in suitable implementations it allows for an effective seal to be created by suction, so that a positive pressure medical gas can be delivered. However it is also applicable to situations where this is not required.

The term airway when used throughout the specification and claims refers to the mouth, the nose or both, as is appropriate for the particular application.

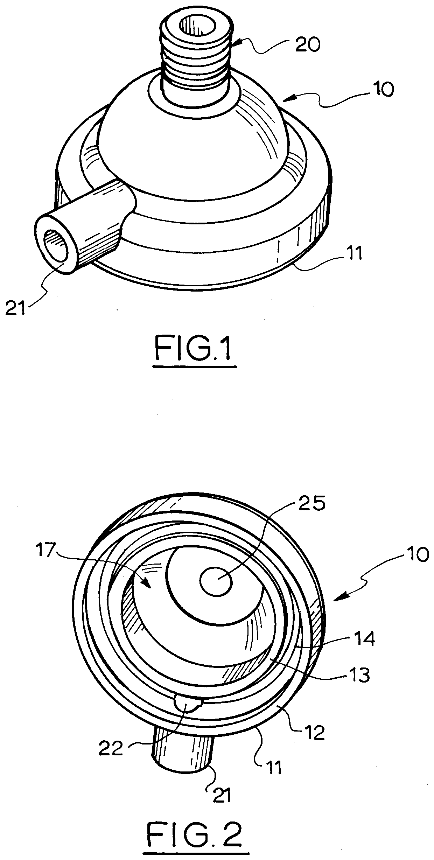

Figures 1 and 2 illustrate an implementation of the present invention suitable for use in neonatal resuscitation. Figure 1 shows the device 10, with a connection 20 to a neonatal resuscitation machine, and more particularly to a source of air. The air may be mixed with some additional oxygen, but for the purposes of simplicity of description, it will be assumed that it is simply air.

Air connection 20 is shown as a conventional input connection for masks for neonatal resuscitation machines, however, it will be appreciated that any suitable connection could be used consistent with the source of air.

Device 10 is generally dome shaped, as is conventional, and is formed from a flexible material, so that device 10 can be deformed so that the seal 11 can engage the skin of the infant, and form a seal around their nose and mouth. Suction connection 21 is shown, again with a shape adapted to allow conventional suction systems to be readily connected.

Figure 2 illustrates the underside of device 10. The seal 11 is formed by outer wall 12, and inner wall 13, which define a channel 14 between them. This channel is connected via opening 22 to connection 21, and hence to a source of suction. Hence, in use, when a source of suction is connected to connection 21, the pressure is lowered in channel 14, and it will adhere to an adjacent surface.

Opening 25 is the open end of connection 20, and opens into the generally open interior chamber 17 of device 10. Hence, in use, a source of suction (not shown) is connected to connection 21, and a source of air (not shown) to connection 20. Device 10 may then be positioned over the mouth and nose of the infant, in the conventional way. However, instead of having to awkwardly hold the mask and the infant, the practitioner can place device 10 in position and allow the suction to seal device 10 to the face of the infant, while attending to the correct support and positioning of the infant. The mask will, under the correct conditions, retain itself in position.

It will be appreciated that while the shape and arrangement illustrated are preferred, it is possible to use the present invention with different shaped masks in suitable applications. The suction chamber on different implementations of the device could extend continuously as shown, or in suitable applications may be discontinuous, so that suction is only at particular points. The shape of the suction chamber could be different to the mask, or it may more closely follow the shape of the individual face, rather than simply being annular. The suction chamber could more closely follow the exact shape of the engagement between the face and the mask. The present invention is not limited in application to a particular shape or dimension for the suction chamber.

As will be described below, the mask is intended to be connected via connection 21 to the low pressure suction connection of an infant resuscitation machine. This will typically operate at around 2.0 kPa.

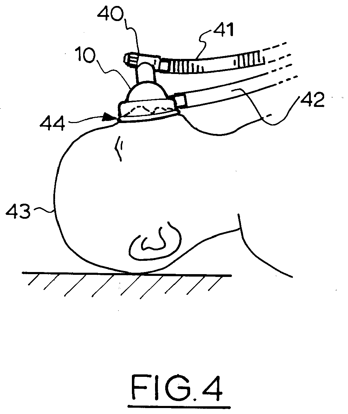

Connection 20 is intended to be connected to conventional T piece 40 (figure 4) and then the resuscitation outlet of an infant resuscitation machine. Typically, an ISO male 15mm fitting is provided. The mask is preferable formed from cast silicone rubber. The preferred material is a medical grade silicone. Other suitable material may be used. The materials are preferable inert and biocompatible. The necessary degree of flexibility and resilience is required to allow the mask to conform to the face of the user. It will be appreciated that Apart from the inclusion of a suction chamber, the mask may be constructed and configured in a similar manner to existing commercially available masks, using conventional manufacturing processes.

The mechanical properties of the mask will be affected not only by the material chosen, but also by wall thicknesses, the overall shape, and the details of construction, as will be understood by those skilled in the art. The walls of the suction chamber must be sufficiently rigid to retain the vacuum, but the mask must also deform to the shape of the face in order to work and seal effectively.

For example, in region 30, it has been determined that additional reinforcing material, i,e, thickness, is required to ensure that the connection 20 remains upright and does not collapse under load. On the other hand, it is important that material in region 31 has sufficient flexibility to allow for the mask to conform to different face shapes.

In the design illustrated, it has been found that it is critical to get the correct balance between flexibility and stiffness, so that the mask can deform without the walls collapsing. It is preferred in this implementation that the Shore hardness of the material be 35 to 45, most preferably 40.

The seal structure 11 includes inner wall 12 and outer wall 13, with the channel 14 between, which in use is evacuated by the suction system. It will be noted that the outer wall extends further in the direction of the airway opening 28. This creates an angle relative to the centre of chamber 17. In a preferred form, this angle is about 30 degrees for an infant. The difference in height provides better conformity to the shape of the face of the patient. The opening 28, for a pre term or full term neonate, varies in preferred width from 20 to 38 mm in this example.

Mask 10 includes a relatively small internal chamber 17. It is preferred to minimise the chamber volume, so that there is as little dead space as possible during operation of the resuscitation system.

The generally circular shape of the device, with the suction connection 21 located peripherally, allows for the mask to be placed so that the connection 21 can be placed in the most convenient position relative to the hose 42 and patient 43 whilst maintaining an effective seal.

The mask must be operated using suction at an appropriate level, balancing firm attachment with avoiding an unnecessarily tight connection. It has been determined in the implementation described that suction pressures between 25 & 45 kPa are suitable. The suction can be provided by connecting to any medical suction device delivering those pressures. Most resuscitation machines have suction units attached. These suction machines generally have control mechanisms on them which will allow for this range to be met.

It will be understood that a particularly advantageous aspect of the illustrated implementation is that the vacuum is only applied to a relatively small annular space 14, rather than a larger volume with mask 10. This minimises the mechanical requirements on the mask, as only limited areas have a relative vacuum on one side, and increased pressure on the other. Hence, there is no tendency for the main chamber 17 to collapse because the activation of the suction system. The present invention is suitable for use with any suitable device. For example, it can be used with devices such as the Resusitaire ® device available from Draeger Australia Pty Ltd, the Optiflow Junior device available from Fisher & Paykel Healthcare, and the Giraffe infant resuscitation device available from GE Healthcare.

It is particularly noted that the above referenced devices include a suction capability, which can be readily employed with the present invention. Alternatively, hospital suction systems may be employed. By suction is meant a system to provide a negative pressure, so that gas is withdrawn and the reduced pressure in the suction chamber relative to the atmosphere provides a retaining force (assuming the pressure reduction is of sufficient size).

A suitable mode of operation for infant resuscitation will now be described. Note that it is not the intention here to fully instruct in the process of resuscitation- this is a matter within the general knowledge of those skilled in the art. The present implementation of the invention is concerned with how the mask is attached to the face of the neonate.

As shown in figure 4, mask 10 is connected to a T piece 40 of conventional construct ion. A connecting flexible pipe 41 provides a connection for positive resuscitation airflows from the resuscitation machine (not shown). Mask 10 is also connected to flexible hose 42 and thereby to the low pressure suction connection of the resuscitation machine.

As shown, the mask 10 is positioned over the airways, both the nose and mouth, of the neonate 43. It can be seen that the mask 10 is well conformed to the face 44 of the neonate 43. Thus, air or air/oxygen mix can be supplied via mask 10 to the neonate 43the suction will retain the mask in place, while the positive pressure medical gas is supplied through mask 10. After initial positioning, the mask will not need to be held in place, the suction will in normal course hold mask 10 in position over the airway.

It will be appreciated that the present invention may also be applied with other means to assist in retaining a mask, for example straps or the like also being employed, in some applications if required.

The connections to suction and to medical gases are shown as conventional connections to neonatal resuscitation machines. It will be appreciated that different connections to the respective portions of the mask, which could be any suitable passage from the corresponding interior structure to the exterior connection or system, could be used. For example, the mask could be integral with the T piece device. The mask could be integrally connected to the appropriate pipes or hoses for connection to a suitable machine.

## Claims

1. A mask (10) for the delivery of a medical gas, the mask(10) including a single shell forming a chamber(17) having an outlet (28), a first passage (20) connecting the chamber(17) to the exterior of the mask, **characterised in that** the mask (10) further includes a sealing recess (11) isolated from the chamber (17) and open to allow engagement with the face of a user, and a second passage (21) connecting the sealing recess (11)to the exterior of the mask, the arrangement being such that operatively the mask (10) may be placed on a user so as to cover the airway of the user, suction may be connected to the second passage (21) so that the sealing recess (11) seals the mask against the face of the user, and a medical gas may be supplied to the first passage (21) for delivery through the outlet (28) to the airway of the user.

2. A mask according to claim 1, wherein the mask (10) is operatively sealed to the face of the user, so that a positive pressure medical gas may be delivered through the chamber (17).

3. A mask according to claim 1 or claim 2, wherein the sealing recess (11) extends around the airway.

4. A mask according to any one of the preceding claims, wherein the sealing recess (11) extends continuously around the periphery of the outlet (28).

5. A mask according to any one of the preceding claims, wherein the mask is formed so as to operatively resiliently deform and accommodate the shape of the face of a user.

6. A mask according to any one of the preceding claims, adapted for infant resuscitation.

7. A mask according to any one of the preceding claims, wherein the mask has a unitary construction.

8. A mask according to claim 7, wherein the mask is formed as a single moulded article.

9. A mask according to claim 4, wherein the sealing recess (11) is formed by an inner wall (13) and an outer wall (14), and the outer wall (14) extends further from the mask than the inner wall (13).

## Patentansprüche

1. Maske (10) zur Zufuhr eines medizinischen Gases, wobei die Maske eine einzelne Schale, die eine Kammer (17) mit einem Auslass (28), einen ersten Durchgang (20), der die Kammer (17) mit dem Äußeren der Maske verbindet, enthält, **dadurch gekennzeichnet, dass** die Maske (10) weiterhin einen Dichtungseinschnitt (11) enthält, der von der Kammer (17) isoliert und offen ist, um ein Ineinandergreifen mit dem Gesicht eines Benutzers zu erlauben, und einen zweiten Durchgang (21), der den Dichtungseinschnitt (11) mit dem Äußeren der Maske verbindet, wobei die Anordnung dergestalt ist, dass die Maske (10) auf einem Benutzer operativ so platziert werden kann, dass sie den Atemweg des Benutzers bedeckt, eine Absaugung an den zweiten Durchgang (21) angeschlossen werden kann, so dass der Dichtungseinschnitt (11) die Maske gegenüber dem Gesicht des Benutzers abdichtet und dem ersten Durchgang (21) ein medizinisches Gas zur Zufuhr durch den Auslass (28) zum Atemweg des Benutzers zugeführt werden kann.

2. Maske gemäß Anspruch 1, wobei die Maske (10) mit dem Gesicht des Benutzers operativ abgedichtet werden kann, so dass durch die Kammer (17) ein medizinisches Gas mit positivem Druck zugeführt werden kann.

3. Maske gemäß Anspruch 1 oder 2, wobei sich der Dichtungseinschnitt (11) um den Atemweg herum erstreckt.

4. Maske gemäß einem der vorangehenden Ansprüche, wobei sich der Dichtungseinschnitt (11) durchgehend um den Umfang des Auslasses (28) herum erstreckt.

5. Maske gemäß einem der vorangehenden Ansprüche, wobei die Maske derart ausgebildet ist, dass sie sich operativ elastisch verformt und dem Gesicht eines Benutzers anpasst.

6. Maske gemäß einem der vorangehenden Ansprüche, die zur Kinder-Reanimation geeignet ist.

7. Maske gemäß einem der vorangehenden Ansprüche, wobei die Maske eine einheitliche Bauweise aufweist.

8. Maske gemäß Anspruch 7, wobei die Maske als einzelner gemoldeter Gegenstand ausgebildet ist.

9. Maske gemäß Anspruch 4, wobei Dichtungseinschnitt (11) durch eine Innenwand (13) und eine Außenwand (14) gebildet ist und sich die Außenwand (14) weiter als die Innenwand (13) von der Maske erstreckt.

## Revendications

1. Masque (10) pour distribuer un gaz médical, le masque (10) comportant une unique coque formant une chambre (17) ayant une sortie (28), un premier passage (20) reliant la chambre (17) à l'extérieure du masque,
masque (10) **caractérisé en ce qu'**
il comprend en outre une cavité de scellement (11) pour isoler la chambre (17) et s'ouvrant pour permettre la mise en contact avec le visage d'un utilisateur, et un second passage (21) reliant la cavité de scellement (11) à l'extérieur du masque, la disposition étant telle qu'en fonctionnement le masque (10) peut être placé sur l'utilisateur pour couvrir les voies respiratoires de l'utilisateur, une aspiration pouvant être reliée au second passage (21) pour que la cavité de scellement (11) réalise l'étanchéité du masque sur le visage de l'utilisateur, et un gaz médical étant fourni au premier passage (21) pour arriver à travers la sortie (28) dans les voies respiratoires de l'utilisateur.

2. Masque selon la revendication 1,
dans lequel
le masque (10) est scellé de manière fonctionnelle au visage de l'utilisateur pour permettre de distribuer un gaz médical à pression positive par l'intermédiaire de la chambre (17).

3. Masque selon la revendication 1 ou 2,
dans lequel
la cavité de scellement (11) entoure les voies respiratoires.

4. Masque selon l'une quelconque des revendications précédentes,
dans lequel
la cavité de scellement (11) s'étend en continu autour de la périphérie de la sortie (28).

5. Masque selon l'une quelconque des revendications précédentes,
dans lequel
le masque est constitué pour se déformer élastiquement en fonctionnement et épouser la forme du visage de l'utilisateur.

6. Masque selon l'une quelconque des revendications précédentes, destiné à la réanimation infantile.

7. Masque selon l'une quelconque des revendications précédentes,
**caractérisé en ce que**
le masque à une structure unitaire.

8. Masque selon la revendication 7,
dans lequel
le masque est une pièce unique moulée.

9. Masque selon la revendication 4,
selon lequel
le moyen de scellement (11) est formé par la paroi intérieure (13) et la paroi extérieure (14), la paroi extérieure (14) s'étendent au-delà de la paroi intérieure (13) par rapport au masque.
